## Europäisches Patentamt

## European Patent Office

## Office européen des brevets

(11) Veröffentlichungsnummer: **0 106 402**
**B1**

(12) **EUROPÄISCHE PATENTSCHRIFT**

(45) Veröffentlichungstag der Patentschrift:
**07.01.88**

(51) Int. Cl.⁴: **A 61 B 6/02**

(21) Anmeldenummer: **83201426.0**

(22) Anmeldetag: **05.10.83**

(54) **Verfahren zum Erzeugen von Schichtbildern.**

(30) Priorität: **09.10.82 DE 3237572**

(43) Veröffentlichungstag der Anmeldung:
**25.04.84 Patentblatt 84/17**

(45) Bekanntmachung des Hinweises auf die Patenterteilung:
**07.01.88 Patentblatt 88/1**

(84) Benannte Vertragsstaaten:
**DE FR GB**

(56) Entgegenhaltungen:
**EP - A - 0 028 864**
**WO - A - 81/01952**
**DE - B - 2 546 785**
**GB - A - 2 105 160**
**US - A - 3 499 146**

(73) Patentinhaber: **Philips Patentverwaltung GmbH,
Wendenstrasse 35 Postfach 10 51 49,
D-2000 Hamburg 1 (DE)**

(84) Benannte Vertragsstaaten: **DE**

(73) Patentinhaber: **N.V. Philips' Gloeilampenfabrieken,
Groenewoudseweg 1, NL-5621 BA Eindhoven (NL)**

(84) Benannte Vertragsstaaten: **FR GB**

(72) Erfinder: **Haaker, Paul, Burgwedeltwiete 11,
D-2000 Hamburg 61 (DE)**
Erfinder: **Klotz, Erhard, Seekamp 110,
D-2083 Halstenbek (DE)**
Erfinder: **Koppe, Reiner, Rugenbergener Weg 3,
D-2000 Hamburg 61 (DE)**
Erfinder: **Linde Rolf, Kamperrege 21, D-2081 Haseldorf
(DE)**
Erfinder: **Möller, Holger, Rübenkamp 84,
D-2000 Hamburg 60 (DE)**

(74) Vertreter: **Hartmann, Heinrich, Dipl.-Ing. et al, Philips
Patentverwaltung GmbH
Wendenstrasse 35 Postfach 10 51 49,
D-2000 Hamburg 1 (DE)**

ACTORUM AG

## Beschreibung

Die Erfindung bezieht sich auf ein Verfahren zum Erzeugen von Schichtbildern eines Objektes, das aus mehreren Strahlenquellenpositionen zur Erzeugung getrennter Einzelbilder durchstrahlt wird, wobei für jedes Einzelbild von der Absorption in seinen Bildpunkten abhängige Messwerte gespeichert werden und für jeden Schichtbildpunkt ein der Absorption entsprechender Bildwert aus den gespeicherten Messwerten der dem Schichtbildpunkt geometrisch zugeordneten Bildpunkte der Einzelbilder abgeleitet wird.

Als Bildpunkt wird dabei ein endliche Abmessungen aufweisender, vorzugsweise quadratischer Bereich eines Einzelbildes bezeichnet, während mit Schichtbildpunkten ein entsprechender Bereich im Schichtbild bezeichnet ist.

Ein solches Verfahren ist aus der US-PS 3 499 146 bekannt. Die Einzelbilder werden dabei mit Hilfe eines Röntgenstrahlers erzeugt, der nacheinander in verschiedene Positionen gebracht wird. Das Strahlenrelief wird von einem Bildwandler, z.B. einem Bildverstärker, aufgenommen, dessen Ausgangsleuchtschirmbild mit Hilfe einer Fernsehkamera abgetastet wird. Das so erzeugte Videosignal stellt die Aufeinanderfolge der den einzelnen Bildpunkten des Einzelbildes zugeordneten Messwerte dar. Es kann in einer digitalen oder in einer analogen Speicheranordnung gespeichert werden. Der Bildwert, der einem Schichtbildpunkt dabei zugeordnet wird, wird aus den Bildpunkten abgeleitet, die dem Schichtbildpunkt geometrisch zugeordnet sind, d.h. denjenigen Bildpunkten, die bei der Aufnahme auf der Verbindungslinie des betreffenden Schichtbildpunktes mit den verschiedenen Strahlenquellenpositionen liegen, indem die Summe der Messwerte gebildet wird. Dabei können zusätzlich Massnahmen getroffen werden, um Werte auszusondern, die wesentlich von den anderen Messwerten abweichen.

Durch die beschriebene Überlagerung der den entsprechenden Bildpunkten der Einzelbilder zugeordneten Messwerte gehen Details, die ausserhalb der Schicht liegen, weniger stark in die Abbildung eines Schichtbildpunktes ein als wenn sie in diesem Schichtbildpunkt liegen würden; sie werden also «verwischt», wie bei den sonstigen Schichtaufnahmeverfahren auch. Bei Objekten mit räumlich relativ gleichmässig verteilter Absorption ergeben sich mit einem solchen Verfahren brauchbare Resultate. Wenn hingegen Objekte aufgenommen werden mit räumlich sehr inhomogen verteilter Absorption, z.B. mit Kontrastmittel gefüllte Arterien bei der Aufnahme der Herzkranzgefässe, werden die ausserhalb einer Schicht befindlichen stark absorbierenden Details in der Schicht mehrfach abgebildet, und zwar um so intensiver, je geringer die Zahl der Strahlenquellenpositionen ist bzw. je geringer die Zahl der Einzelbilder ist. Diese Artefakte beeinträchtigen die Auswertung solcher Schichtbilder erheblich.

Es ist Aufgabe der vorliegenden Erfindung, ein Verfahren der eingangs genannten Art so auszugestalten, dass brauchbare Schichtbilder derartiger Objekte auch mit Hilfe von wenigen Einzelbildern erzeugt werden können.

Diese Aufgabe wird erfindungsgemäss dadurch gelöst, dass der Bildwert durch den bzw. durch die Messwert(e) gebildet wird, der bzw. die der geringsten Absorption entspricht (entsprechen).

Die Erfindung basiert auf folgender Überlegung.

Wenn ein Objekt nur aus einer dünnen Schicht bestehen würde, dann wäre die Absorption bzw. die Intensität in allen einem bestimmten Schichtbildpunkt zugeordneten Bildpunkten der Einzelbilder einander gleich und nur durch die Absorption im Schichtbildpunkt bestimmt. In realen Objekten befinden sich aber in der Regel beiderseits dieser Schicht auch noch absorbierende Strukturen. Infolgedessen sind die Intensitäten in den genannten Bildpunkten niedriger als in dem erstgenannten Fall, und sie weichen in der Regel voneinander ab. Die Abweichungen der Intensitäten von dem erstgenannten Fall sind um so ausgeprägter, je mehr die Strukturen ausserhalb der Schicht absorbieren. Anders ausgedrückt: der grösste Intensitätswert ist am wenigsten durch absorbierende Strukturen ausserhalb der Schicht beeinflusst worden und ist daher am ehesten ein Mass für die Absorption in dem Schichtbildpunkt. Wenn man daher nur diesen Wert als Bildwert für den Schichtbildpunkt zugrundelegt, werden die durch absorbierende Strukturen ausserhalb der Schicht bedingten Artefakte in dem Bild dieser Schicht weitgehend unterdrückt. Man kann daher mit dem erfindungsgemässen Verfahren aus nur vier Einzelbildern (Strahlenquellenpositionen) brauchbare Schichtaufnahmen erzeugen.

Eine Weiterbildung des erfindungsgemässen Verfahrens sieht vor, dass anschliessend der einem Schichtbildpunkt zugeordnete Bildwert von den Messwerten der diesem Schichtbildpunkt geometrisch zugeordneten Einzelpunkte subtrahiert wird und dass die so berechneten Messwerte bei der Erzeugung weiterer Schichtbilder zugrundegelegt werden. Diese Weiterbildung geht von folgender Überlegung aus: Wenn der Messwert, der der grössten Intensität bzw. der geringsten Absorption entspricht, im wesentlichen durch die Absorption im Schichtbildpunkt bestimmt ist, dann muss auch in den Messwerten der anderen Bildpunkte, die dem Schichtbildpunkt geometrisch zugeordnet sind, ein entsprechender Anteil vorhanden sein. Dieser Anteil wird durch die Subtraktion beseitigt (vorausgesetzt die Messwerte entsprechen dem Logarithmus, der Strahlungsintensität in den jeweiligen Bildpunkten). Infolgedessen kann bei der Erzeugung von anderen Schichtbildern des Objektes dieser Anteil die Bildwerte der Schichtbildpunkte der anderen Schicht nicht mehr beeinflussen, was die Genauigkeit erhöht.

Eine Vorrichtung zur Durchführung des Verfahrens, die ausgeht von einer Strahlenquellenanordnung zum Erzeugen mehrerer Einzelbilder des Objektes aus je einer Strahlenquellenposition mit einer Bildwandleranordnung zur Umwandlung jedes Einzelbildes in elektrische Signale, einer Speicheranordnung zum Speichern der den Bildpunkten der Einzelbilder zugeordneten Signale, Mitteln zum Aufrufen der zu den einem Schichtbildpunkt geometrisch zugeordneten Bildpunkte gehörigen Signale aus der Speicheranordnung und zum Zuführen eines daraus abgeleiteten Wertes an eine Bildwiedergabeeinheit, ist dadurch gekennzeichnet, dass die aus der Speicheranordnung aufgerufenen Signale einem Extremwertdekoder zugeführt werden, dessen Ausgangssignal dasjenige aufgerufene Signal bildet, das der geringsten Absorption entspricht. Eine Weiterbildung dieser Vorrichtung besteht darin, dass die Speicheranordnung eine der Zahl der Strahlenquellenposition gleiche Zahl von Speicherbereichen aufweist, die jeweils die dem Bildpunkt eines Einzelbildes zugeordneten Signalwerte speichern und die gleichzeitig und unabhängig voneinander adressierbar sind und dass die Bildwiedergabeeinheit das Schichtbild punktweise ausgibt und so mit dem Speicherbereich synchronisiert ist, dass jeweils der Schichtbildpunkt wiedergegeben wird, dessen geometrisch zugeordnete Bildpunkte gerade aus dem Speicherbereich aufgerufen sind. Dabei ist eine Echtzeitverarbeitung möglich, d.h. die Bildwerte der Schichtbildpunkte werden genauso schnell gebildet wie sie von der Bildwiedergabeeinheit, z.B. einem Fernsehmonitor, wiedergegeben werden.

Die Erfindung wird nachstehend anhand der Zeichnung näher erläutert. Es zeigen

Fig. 1 ein Diagramm zur Erläuterung des der Erfindung zugrundeliegenden Prinzips,

Fig. 2 eine Vorrichtung zur Erzeugung der Einzelbilder und zum Speichern der Messwerte, die den Bildpunkten, aus denen sich die Einzelbilder zusammensetzen, zugeordnet sind,

Fig. 3 einen Ausschnitt des mit der Anordnung nach Fig. 2 erzeugten Strahlenreliefs, und

Fig. 4 eine Vorrichtung, die aus den gespeicherten Messwerten die den Bildwerten der Schichtpunkte zugeordneten Bildwerte ermittelt und darstellt.

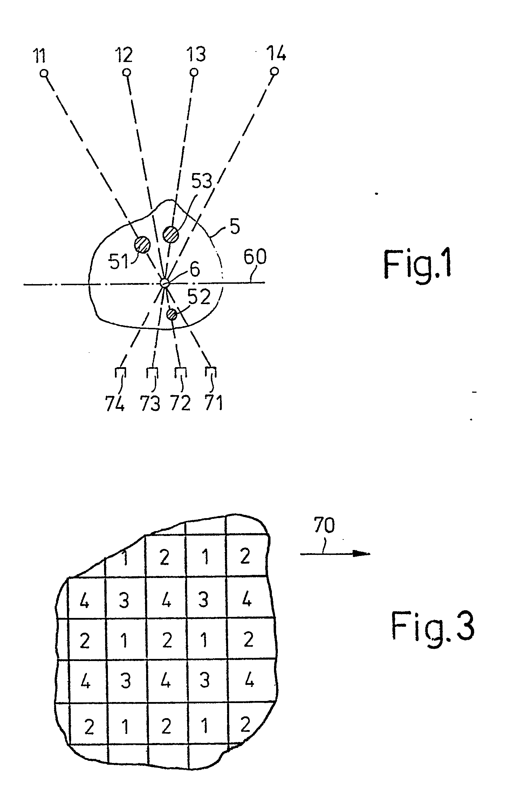

Fig. 1 zeigt ein Objekt 5, das von mehreren Strahlenquellenpositionen bzw. mehreren Strahlenquellen 11, 12, 13 und 14 mit gleicher Intensität bestrahlt wird. Dabei wird u.a. auch der Punkt bzw. das Volumenelement 6 in einer Schicht 60 durchstrahlt. Die Intensität der den Schichtbildpunkt 6 durchsetzenden und von den Strahlenquellen 11...14 ausgehenden Strahlen wird durch die hinter dem Objekt 5 angeordneten Wandler 71...74 gemessen. Die strahlenabsorbierenden Strukturen – z.B. mit Kontrastmittel gefüllte Arterien – sind in dem Objekt an einigen Stellen konzentriert, u.a. in den Bereichen 51, 52 und 53, die auf den den Schichtbildpunkt 6 durchstossenden Strahlen der Strahlenquellen 11, 12 und 13 liegen. Infolgedessen messen die Wandler 71, 72 und 73, die diesen Strahlenquellen zugeordnet sind, eine wesentlich geringere Intensität (bzw. eine stärkere Absorption) als der Wandler 74, der die Strahlung der Strahlenquelle 14 erfasst, weil die Intensität des von diesem Wandler gemessenen, von der Strahlenquelle 14 emittierten Strahles praktisch nicht durch ausserhalb der Schicht 60 liegende stark absorbierende Strukturen beeinflusst wird.

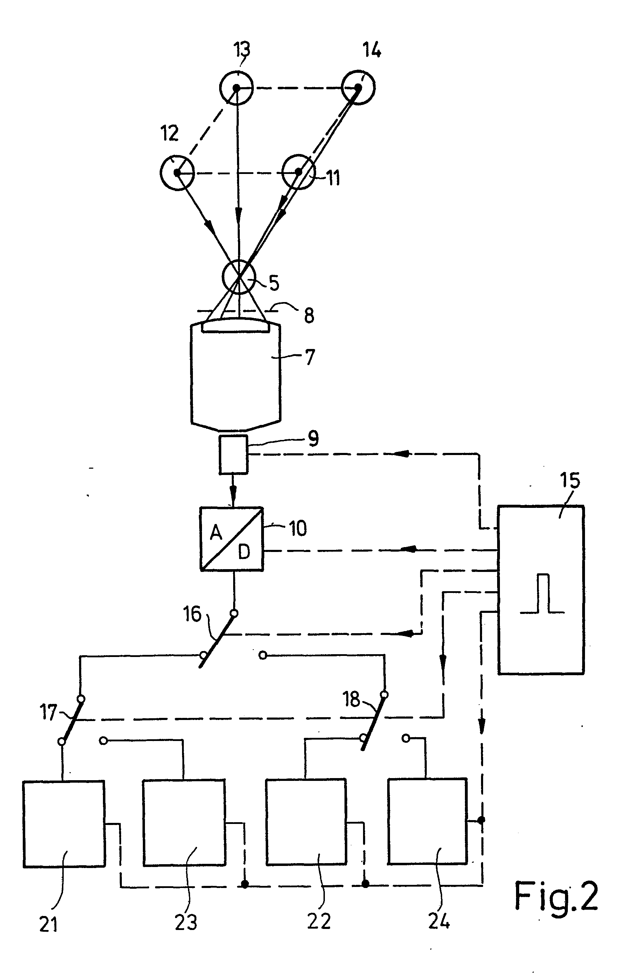

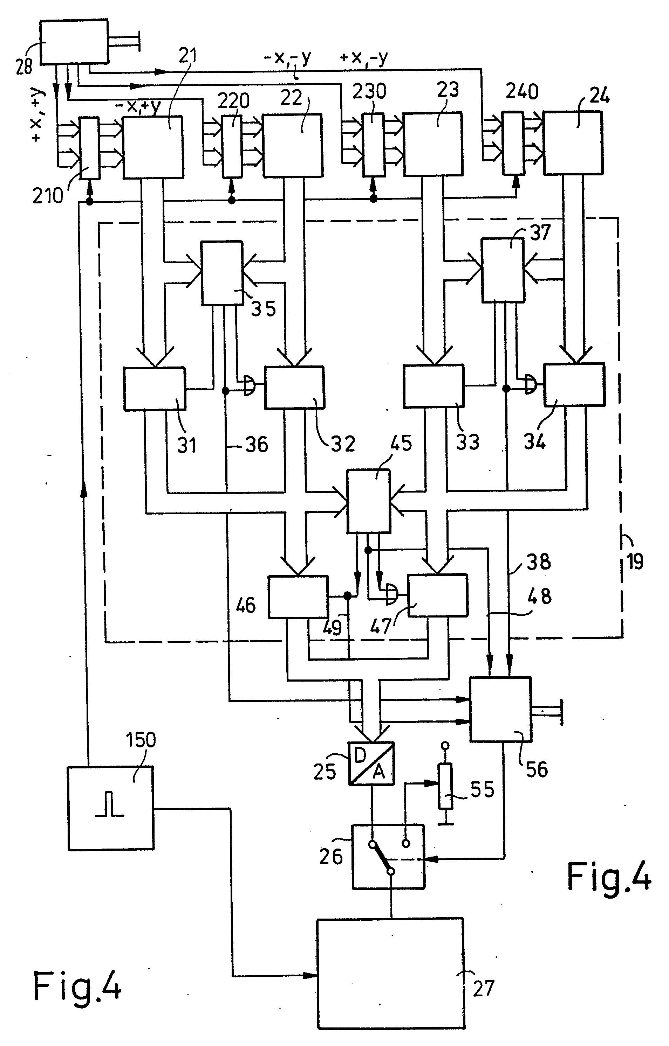

Würde man, wie bei den bisher üblichen Verfahren, der dem Schichtbildpunkt 6 zugeordneten Bildwert durch Überlagerung der von den Wandlern 71...74 erzeugten Messwerte erzeugen, dann ergäbe sich ein Bildwert der einer starken Absorption im Schichtbildpunkt 6 entsprechen würde, selbst dann, wenn die tatsächliche Absorption in diesem Punkt nur sehr gering wäre. Mit anderen Worten, es würden die Strukturen 51, 52 und 53, die ausserhalb der Schicht liegen, in das Bild der Schicht hineinprojiziert. Diese Artefakte werden aber vermieden, wenn lediglich der von dem Wandler 74 gelieferte Messwert, der der geringsten Absorption bzw. der grössten Intensität entspricht, als Bildwert im Schichtbildpunkt 6 ausgegeben wird. Die ausserhalb der Schicht befindlichen Strukturen 51, 52 und 53 haben dann keinen Einfluss auf den Bildwert des Schichtbildpunktes 6, d.h. sie werden nicht in der Schicht abgebildet. Dieses Verfahren liegt der in Fig. 2 und insbesondere in Fig. 4 dargestellten Vorrichtung zugrunde.

Die Vorrichtung nach Fig. 2 enthält die vier Strahlenquellen 11...14, vorzugsweise Röntgenstrahler, die so angeordnet sind, dass sich ihre Brennflecke auf den Eckpunkten eines Quadrates befinden. Die vier Strahlenquellen können im Betrieb Strahlenbündel aussenden, die in der Zeichnung durch eine Gerade veranschaulicht sind, die jedoch das gesamte Objekt 5 erfassen und sich in einer zur Ebene des erwähnten Quadrates parallelen Ebene, die vorzugsweise auch das Objekt 5 schneidet, praktisch decken. Die Strahlenquellen emittieren Strahlung derselben Intensität mit gleicher Strahlungsdauer.

Wie aus den Erläuterungen zu Fig. 1 hervorgeht, ist es für die Erfindung wesentlich, dass die Einzelbilder getrennt sind, d.h. die Bildpunkte eines Einzelbildes dürfen nur von einem der vier Strahler erzeugt werden. Grundsätzlich liesse sich dies dadurch erreichen, dass die Strahler nacheinander eingeschaltet werden (oder ein Strahler nacheinander an die entsprechenden Punkte gebracht und eingeschaltet wird), wobei jedes Einzelbild gespeichert wird, bevor das nächste Einzelbild angefertigt wird. Dabei würde das Objekt 5 also zu unterschiedlichen Zeitpunkten durchstrahlt, was bei sich bewegenden bzw. bewegten Objekten zu Unschärfen führen könnte. Es wäre auch möglich, die Wandleranordnung, die das räumliche Strahlungsintensitätsmuster in elektrische Messwerte umsetzt, so weit vom Objekt 5 entfernt anzuordnen, dass die durch die vier Strahler erzeugten Strahlenreliefs nebeneinander auf der Eingangsfläche der Wandleranordnung Platz fänden. Dabei müsste jedoch die Strahlendosis vergrössert werden und die Eingangsfläche müsste entsprechend gross sein.

Zur Erzeugung getrennter – wenn auch ineinander verschachtelter – Einzelbilder wird daher, wie aus der DE-PS 2 546 785 bekannt, ein zweidimensionales Raster 8 verwendet, das in einer zur Ebene des erwähnten Quadrates parallelen Ebene liegt und das so viele quadratische Öffnungen aufweist, wie das Einzelbild Bildpunkte haben soll, z.B. 256 × 256 (oder 512 × 512). Das Raster 8 ist so zwischen dem Objekt 5 und einer Wandleranordnung 7 angeordnet, dass durch jede Öffnung hindurch von den vier Strahlern 11 . . . 14 vier Bildpunkte bestrahlt werden, wobei diese vier Bildpunkte neben anderen Bildpunkten liegen (und sich nicht mit diesen überlappen), die durch eine benachbarte Öffnung bestrahlt werden.

Auf der Eingangsfläche des Bildwandlers 7 ergibt sich daher ein Bild, von dem ein Ausschnitt in Fig. 3 dargestellt ist. Dabei sind mit 1, 2, 3 bzw. 4 Bildpunkte bezeichnet, die von den Strahlern 11, 12, 13 bzw. 14 durch die verschiedenen Öffnungen in dem Raster 8 hindurch erzeugt werden. Wie man aus Fig. 3 erkennt, werden die Bildpunkte jeder zweiten Zeile abwechselnd von den Strahlern 11 und 12 erzeugt, während die Bildpunkte der dazwischenliegenden Zeilen von den Strahlern 13 und 14 erzeugt werden.

Der Bildwandler kann grundsätzlich durch eine Matrix von Halbleiterdetektoren gebildet werden, deren Abmessungen den Abmessungen der Bildpunkte entsprechen und deren Zahl viermal so gross ist wie die Zahl der Bildpunkte eines Einzelbildes, so dass jeder der von den vier Strahlenquellen erzeugten Bildpunkte von einem gesonderten Detektor erfasst werden könnte. Eine solche Detektoranordnung ist jedoch noch relativ aufwendig. Deshalb wird an ihrer Stelle ein Bildverstärker 7 verwendet, auf dessen Eingangsschirm die den Einzelbildern entsprechenden Intensitätsreliefs getrennt, jedoch gemäss Fig. 3 ineinanderverschachtelt, auftreffen. Am Ausgang des Bildverstärkers erscheint das Eingangsbild verkleinert, jedoch mit wesentlich grösserer Intensität. Dieses Ausgangsbild wird mit Hilfe einer Fernsehkamera 9 aufgenommen. Wenn die Zeilenrichtung, mit der in der Fernsehkamera 9 das Bild auf dem Ausgangsschirm des Bildverstärkers 7 abgetastet wird, in Richtung des Pfeiles 70 in Fig. 3 gelegt ist, und wenn die Zahl der Zeilen dieser Fernsehkamera doppelt so gross ist wie die Zahl der Bildpunktzeilen in einem Einzelbild (bei 256 × 256 Bildpunkten pro Einzelbild also 512 Zeilen; bei 512 × 512 Bildpunkten 1024 Zeilen), wird mit jeder Zeile eine der aus Fig. 3 ausschnittsweise ersichtlichen Bildpunktreihen abgetastet.

Das von der Fernsehkamera erzeugte Videosignal wird während einer Zeile doppelt so häufig abgetastet wie Bildpunkte in den Reihen eines Einzelbildes vorhanden sind, d.h. also bei den angegebenen Beispielen 512 bzw. 1024 mal, und mit Hilfe eines Analog-Digital-Wandlers 10 in eine Binärzahl bzw. digitales Datenwort umgesetzt, das somit ein Mass für die Intensität bzw. die Absorption in dem betreffenden Bildpunkt darstellt. Zweckmässigerweise wird das Videosignal vor der Digitalisierung logarithmiert bzw. sein Dynamikbereich durch einen geeigneten Compander eingeschränkt, wobei darauf geachtet werden muss, dass die Binärzahlen umkehrbar eindeutig der Absorption bzw. der Strahlungsintensität in den einzelnen Bildpunkten zugeordnet bleiben.

Die Anordnung umfasst vier Digitalspeicher 21 . . . 24, von denen jeder einem der Strahler 11 . . . 14 zugeordnet ist, d.h. jeder dieser Speicher speichert die Datenworte, die den vom zugehörigen Strahler 11 . . . 14 erzeugten Bildpunkten zugeordnet sind. Zu diesem Zweck ist die Ausgangsleitung des Analog-Digital-Wandlers (in Wirklichkeit ein Datenbus) über einen als Umschalter dargestellten ersten Demultiplexer 16 mit den Eingängen zweier weiterer Demultiplexer 17 und 18 verbunden. Die Ausgänge des Demultiplexers 17 sind mit den Dateneingängen der Speicher 21 und 23 verbunden, während die Ausgänge des Demultiplexers 18 mit den Dateneingängen der Speicher 22 und 24 verbunden sind.

Ein Taktgenerator 15, der auch die Synchronsignale für die Fernsehkamera 9 erzeugt, schaltet den Demultiplexer 16 im gleichen Takt um, in dem der Analog-Digital-Wandler unter dem Einfluss eines ebenfalls von dem Taktgenerator 15 gelieferten Taktsignals Datenworte erzeugt. Infolgedessen werden die vom Analog-Digital-Wandler 10 erzeugten Datenworte über den Demultiplexer 16 abwechselnd dem Speicher 21 und dem Speicher 22 zugeführt, solange die Demultiplexer 17 und 18 ihre aus der Zeichnung ersichtliche Schaltstellung haben. Wenn die dabei erzeugten Datenworte der Absorption bzw. der Intensität in der Bildpunktreihe 1, 2, 1, 2, usw. (vgl. Fig. 3) entsprechen, werden auf diese Weise die den Bildpunkten 1 (Fig. 3) zugeordneten Datenworte in den Speicher 21 und die den Bildpunkten 2 zugeordneten Datenworte in den Speicher 22 übernommen. Die Adressierung dieser Speicher wird durch den Taktgenerator 15 mit der halben Taktfrequenz umgeschaltet wie der Analog-Digital-Wandler 10.

Bei (bzw. unmittelbar vor) Beginn der nächsten Fernsehzeile werden die Demultiplexer 17 und 18 in die in Fig. 2 nicht gezeigte Schaltstellung umgeschaltet, so dass dann die Datenworte, die den Bildpunkten 3 bzw. 4 in Fig. 3 zugeordnet sind, in die Speicher 23 und 24 eingelesen werden. Bei der nächsten Zeile werden die Demultiplexer 17 und 18 wieder in die dargestellte Schaltstellung zurückgebracht usw. Wenn die Fernsehkamera 9 das gesamte Ausgangsschirmbild des Bildverstärkers 7 in ein Videosignal umgesetzt hat, ist in jedem der Speicher 21 . . . 24 die Intensitäts- bzw. Absorptionsverteilung der von den vier Röntgenstrahlern 11 . . . 14 erzeugten Einzelbilder gespeichert.

Zur Erzeugung eines Bildes derjenigen Schicht, in der sich die von den vier Röntgenstrahlern 11 . . . 14 emittierten Strahlenbündel decken, wird aus jedem der Speicher 21, 22, 23 und 24 das darin jeweils zuerst gespeicherte Datenwort ausgelesen und über entsprechende Busleitungen den vier Eingängen eines Extremwertdekoders 19 zugeführt. Dieser schaltet dasjenige Datenwort an seinen vier Eingängen, das dem grössten Binärwert und mithin der grössten Intensität entspricht, zu seinem einzigen Ausgang durch, an den ein Digital-Analog-Wandler 25 angeschlossen ist, der über eine Umschalteranordnung 26 mit einem Fernsehmonitor 27 verbunden ist. Anschliessend werden vier weitere Datenworte aus den Speichern 21 ... 24 gleichzeitig ausgelesen, und zwar jeweils in der Reihenfolge in der sie in dem betreffenden Speicher eingelesen wurden. Der Taktgenerator 150 liefert den Adressenzählern 210, 220, 230 und 240 die den Speichern 21 ... 24 zugeordnet sind, Taktimpulse und ebenso den Ablenkgeneratoren des Fernsehmonitors 27, so dass diese derart synchronisiert sind, dass am Videoeingang des Fernsehmonitors 27 die in Analogsignale umgesetzten Datenworte mit der Geschwindigkeit erscheinen, wie sie für die Erzeugung eines die Schicht darstellenden Fernsehbildes benötigt werden.

Wenn Bilder anderer Schichten erzeugt werden sollen, müssen jeweils die Datenworte aufgerufen werden, die zu den Bildpunkten gehören, die dem jeweils zu erzeugenden Schichtbildpunkt geometrisch zugeordnet sind. Diese Datenworte sind in den Speichern 21 ... 24 nicht mehr unter derselben Adresse gespeichert. Beim Aufrufen der Datenworte müssen die Adressen daher entsprechend modifiziert werden.

Wenn Einzelbilder mit z.B. 256 × 256 Bildpunkten verarbeitet werden, ist diese Modifikation der Adressen verhältnismässig einfach, wenn davon ausgegangen wird, dass die Lage eines Bildpunktes innerhalb einer Bildpunktreihe bzw. -zeile durch die letzten acht Binärstellen der Adresse bestimmt ist und dass die Lage einer Bildpunktreihe innerhalb eines Einzelbildes durch die ersten acht Binärstellen des Adressenwortes bestimmt ist. Die letzten acht Binärstellen der Speicher 21 und 24, die den Strahlern 11 und 14 zugeordnet sind, deren Verbindungslinie senkrecht zur Richtung der Bildpunktreihe verläuft, müssen dabei um den gleichen Betrag (z.B. $+x$) geändert werden. Dasselbe gilt für die Änderung der letzten acht Binärstellen der Adressenworte für die Speicher 22 und 23, jedoch muss diese Änderung der erstgenannten entgegengesetzt gleich sein ($-x$). Die ersten acht Binärstellen der Adressenworte für die Speicher 21 und 22 müssen um den gleichen Betrag ($+y$) geändert werden. Dasselbe gilt für die Speicher 23, 24, jedoch mit entgegengesetztem Vorzeichen ($-y$).

Der erforderliche Adressenversatz lässt sich leicht realisieren, wenn die Adresszähler 210 ... 240 als voreinstellbare Binärzähler ausgebildet sind, denen der Voreinstellwert (x, y) in der beschriebenen Weise vorgegeben wird. Dies kann mit Hilfe einer vom Benutzer z.B. über eine Tastatur oder eine analoge Eingabeeinrichtung bedienbaren Schichtwahlvorrichtung 27 erfolgen. Auf diese Weise ist es möglich, aus den Einzelbildern nachträglich Bilder beliebiger Schichten zu erzeugen, deren Lage eindeutig durch die Parameter x, y bestimmt ist.

Es wird nunmehr der Extremwertdekoder 19 näher beschrieben. Er erhält vier Bustreiber 31 ... 34, deren Eingänge jeweils mit den Datenausgängen des ihnen zugeordneten Speichers 21 ... 24 über eine Busleitung verbunden sind. Das Eingangssignal eines solchen Bustreibers 31 ... 34 erscheint (verstärkt) an seinem Ausgang, wenn an seinem Freigabeeingang ein entsprechendes Signal anliegt. Die Freigabeeingänge der Busleitungstreiber 31 und 32 werden durch einen Vergleicher 35 gesteuert, dessen Eingänge über Busleitungen mit den Ausgängen der Speicher 21 und 22 verbunden sind. Der Vergleicher hat zwei Ausgänge, die signalisieren, ob die am Ausgang des Speichers 21 bzw. die am Ausgang des Speichers 22 anliegende Binärzahl grösser ist. Ein dritter Ausgang 36 zeigt an, dass beide Binärzahlen gleich gross sind. Die drei Ausgänge des Vergleichers sind direkt bzw. über eine Oder-Verknüpfung so mit den Freigabeeingängen der Busleitungstreiber 31 und 32 verbunden, dass der Busleitungstreiber 31 freigegeben wird, wenn die vom Speicher 21 gelieferte Binärzahl grösser ist als die Binärzahl am Ausgang des Speichers 22, und dass die Busleitungstreiber 32 freigegeben wird, wenn die Binärzahl am Ausgang des Speichers 22 gleich ist oder grösser als die Binärzahl am Ausgang des Speichers 21. An den beiden miteinander verbundenen Ausgängen der Busleitungstreiber 31 und 32 liegt daher jeweils die grössere der beiden Binärzahlen an.

Auf gleiche Weise sind die Busleitungstreiber 33 und 34 durch einen Komparator 37 so gesteuert, dass an ihren miteinander verbundenen Ausgängen jeweils die grössere der beiden Binärzahlen erscheint bzw. – falls beide Binärzahlen gleich sind – diese Binärzahl. Der Ausgang 38 des Vergleichers 37 signalisiert, dass beide Binärzahlen gleich sind.

Die miteinander verbundenen Ausgänge der Busleiter 31, 32 einerseits und der Busleitungstreiber 33 und 34 andererseits sind mit den Eingängen eines dritten Vergleichers 45 verbunden sowie mit je einem weiteren Busleitungstreiber 46 bzw. 47, die von dem Vergleicher 45 wiederum so gesteuert werden, dass an ihren miteinander und mit dem Eingang des Digital-Analog-Wandlers 25 verbundenen Ausgängen jeweils die grössere der beiden Binärzahlen erscheint bzw. – falls die beiden Binärzahlen einander gleich sind – eine dieser Binärzahlen. Auf diese Weise wird dem Digital-Analog-Wandler 25 jeweils die grösste der an den Ausgängen der vier Speicher 21 ... 24 anliegenden Binärzahlen zugeführt bzw., falls mehrere Binärzahlen den Maximalwert haben, eine dieser Binärzahlen.

Es leuchtet ein, dass der Extremwertdekoder 19 an seinem Ausgang jeweils die kleinste Binärzahl liefern muss, wenn das Videosignal in den Speichern 21 ... 24 in einer solchen Form gespeichert ist, dass der kleinsten Absorption bzw. der grössten Intensität die kleinsten Binärzahlen zugeordnet sind. In diesem Fall müssen lediglich die Freigabeeingänge der zwei von einem Vergleicher gesteuerten Busleitungstreiber (z.B. 31, 32) miteinander vertauscht werden.

Wenn in drei der vier einem Schichtbildpunkt (z.B. 6 in Fig. 1) geometrisch zugeordneten Bildpunkten eine Intensität gemessen wird, die wesentlich kleiner ist als die Intensität im vierten Bildpunkt, dann kann dies zwei Ursachen haben:

a) Drei Strahlenquellen durchstrahlen ausser dem Schichtbildpunkt 6 noch weitere absorbierende Strukturen (51 . . . 53 in Fig. 1), die ausserhalb der Schicht liegen.

b) Es liegt ein Messfehler vor.

Das bedeutet also, dass für den Fall, dass nur ein Maximalwert gefunden wird, immer eine gewisse Unsicherheit besteht, ob dieser Messwert tatsächlich durch die Absorptionsverhältnisse im Schichtbildpunkt bestimmt ist oder durch einen Messfehler. Wenn hingegen zwei zumindest annähernd gleich grosse Maximalwerte vorliegen, sind Messfehler unwahrscheinlicher. Dies gilt noch ausgeprägter, wenn drei oder gar vier annähernd gleich grosse Maximalwerte vorliegen. Das bedeutet, dass sich das Schichtbild aus Bildwerten zusammensetzt, bei denen die Wahrscheinlichkeit, dass der betreffende Wert richtig gemessen worden ist, von Schichtbildpunkt zu Schichtbildpunkt schwanken kann.

Schichtbildpunkte, denen nur ein (oder zwei bzw. drei) Maximalwert(e) zugeordnet werden kann (können) und die somit unsicherer sind als Schichtbildpunkte, denen mehr Maximalwerte zugeordnet werden können, können mit Hilfe des Umschalters 26 ausgetastet werden, der den Videoeingang des Fernsehmonitors 27 immer dann mit einer geeigneten z.B. an einem Potentiometer 55 abgreifbaren Spannung (anstatt mit dem Ausgang des Digital-Analog-Wandlers) verbindet, wenn weniger als zwei (oder drei bzw. vier) Maximalwerte vorhanden sind. Der Umschalter 26 wird von einer logischen Schaltung 56 gesteuert, die die Signale auf den Leitungen 36, 38 und der entsprechenden Leitung 48 der Komparatoren 35, 37, 45 und einer der beiden anderen Ausgangsleitungen des Vergleichers 45 miteinander verknüpft. Der Signalzustand auf diesen vier Leitungen definiert eindeutig, ob ein, zwei, drei oder vier Maximalwerte vorhanden sind. Zweckmässigerweise ist die logische Verknüpfungsschaltung 56 so ausgebildet, dass der Benutzer durch eine geeignete Eingabeeinrichtung vorgeben kann, wie viele der vier von den Speichern gelieferten Binärzahlen dem Maximalwert entsprechen müssen. Da es dabei, wie schon eingangs erläutert, nur darauf ankommt, dass die Binärzahlen dem Maximalwert wenigstens annähernd entsprechen, brauchen den Komparatoren 35, 37 und 45 nicht alle Binärstellen einer Binärzahl zugeführt zu werden, sondern nur jeweils die höchstwertigen.

Auf diese Weise ist es dem Benutzer möglich, das Mass der Sicherheit für die einzelnen Schichtbildpunkte vorzugeben.

Aus dem Vorstehenden ergibt sich, dass aus den vier durch gleichzeitiges Einschalten der Röntgenstrahler 11 . . . 14 aufgenommenen Einzelbildern Schichtbilder hergestellt werden können, deren Lage vorgebbar ist und bei dem auch die Sicherheit, dass Schichtbildpunkte nicht auf Messfehlern beruhen, vorgegeben werden kann. Die Zeitdauer, die erforderlich ist, um dieses Schichtbild zu erzeugen, entspricht der Dauer zweier Fernsehbilder und der Einschaltzeit. Es ist möglich, die vier Röntgenstrahler auch periodisch einzuschalten, um dynamische Prozesse aufzunehmen und davon Schichtaufnahmen unter Durchleuchtung herzustellen («Tomoskopie»). Wenn weiterhin die dabei erzeugten Fernsehsignale der Bildserien auf einen geeigneten Speicher (z.B. Magnetband) gespeichert werden, ist eine nachträgliche Verarbeitung möglich. Es ist auch möglich, die Einzelbilder, die nacheinander von z.B. zwei nebeneinanderliegenden Röntgenstrahlern erzeugt werden, als Bilder eines Stereo-Videofilms zu verarbeiten. Jede Projektionsrichtung kann als Videofilm dargestellt werden. Natürlich kann jedes Bild für sich als Standbild gezeigt werden.

Besonders geeignet ist das beschriebene Schichtverfahren für Bilder, wie sie in der digitalen Subtraktionsangiographie anfallen. Um diese Subtraktion im Zeit- oder Energiemode durchzuführen, kann die beschriebene Hardware aus Fig. 4 benutzt werden. Für die Aufnahme im Energiemode, müssen die Röhren hintereinander mit verschiedenen Spannungen betrieben werden.

Es ist auch denkbar, dass man diese Verfahren mit Film (denkbar ist auch Futji Platte) durchführt. Besonders vorteilhaft ist es, dass der Bildträger planar ist und demzufolge keine Bildgeometriefehler auftreten. Die Filmbilder können mit bekannten Filmabtastern (Optronix-Photoscan) digitalisiert werden. Geeignet ist aber auch eine Linsen-Matrix (Linsen angeordnet wie Röntgenröhren), die die verschiedenen Perspektivbilder nacheinander – über Photoverschlüsse – auf eine Fernsehkamera projiziert. Die Weiterverarbeitung erfolgt dann nach Fig. 2. (LinsenMatrix zur Dekodierung von Schichten ist beschrieben in der deutschen Patentanmeldung P 2 746 035.1). Grundsätzlich können die Primärbilder, bevor sie z.B. zu Schichtbildern weiterverarbeitet werden, nach bekannten Verfahren vorprozessiert werden (z.B. Hochpassfilterung, Differenzierung usw.). Zusätzlich zu den Aufnahmeröhren (4) kann z.B. zum Positionieren des Patienten und zum Katheterisieren von Gefässen eine Röhre in der Mitte vorgesehen sein.

## Patentansprüche

1. Verfahren zum Erzeugen von Schichtbildern eines Objektes, das aus mehreren Strahlenquellenpositionen zur Erzeugung getrennter Einzelbilder durchstrahlt wird, wobei für jedes Einzelbild von der Absorption in seinen Bildpunkten abhängige Messwerte gespeichert werden und für jeden Schichtbildpunkt ein der Absorption entsprechender Bildwert aus den gespeicherten Messwerten der dem Schichtbildpunkt geometrisch zugeordneten Bildpunkte abgeleitet wird, dadurch gekennzeichnet, dass der Bildwert durch den bzw. durch die Messwert(e) gebildet wird, der bzw. die der geringsten Absorption entspricht (entsprechen).

2. Verfahren nach Anspruch 1, dadurch gekennzeichnet, dass anschliessend der einem Schichtbildpunkt zugeordnete Bildwert von den Messwerten der diesem Schichtbildpunkt geometrisch zugeordneten Einzelbildpunkte subtrahiert wird und dass die so berechneten Messwerte bei der Erzeugung weiterer Schichtbilder zugrundegelegt werden.

3. Verfahren nach Anspruch 1, dadurch gekennzeichnet, dass für jeden Schichtbildpunkt die Zahl derjenigen geometrisch zugeordneten Einzelbildpunkte ermittelt wird, deren Messwerte wenigstens annähern dem Maximalwert der Absorption entsprechen.

4. Vorrichtung mit einer Stahlenquellenanordnung (11 . . . 14) zur Erzeugung mehrerer Einzelbilder des Objektes (5) aus je einer Strahlenquellenposition mit einer Bildwandleranordnung (7, 9) zur Umwandlung jedes Einzelbildes in elektrische Signale, einer Speicheranordnung (21 . . . 24) zum Speichern der den Bildpunkten der Einzelbilder zugeordneten Signale, mit Mitteln (28; 210 . . . 240; 150) zum Aufrufen der zu den einem Schichtbildpunkt geometrisch zugeordneten Bildpunkten gehörigen Signale aus der Speicheranordnung (21 . . . 24) und zum Zuführen eines daraus abgeleiteten Wertes an eine Bildwiedergabeeinheit (17), dadurch gekennzeichnet, dass die aus der Speicheranordnung (21 . . . 24) aufgerufenen Signale der Bildwiedergabeeinheit über einen Extremwertdekoder (19) zugeführt werden, dessen Ausgangssignal dasjenige aufgerufene Signal bildet, das der geringsten Absorption entspricht.

5. Vorrichtung nach Anspruch 4, dadurch gekennzeichnet, dass die Speicheranordnung eine der Zahl der Strahlenquellenpositionen gleiche Zahl von Speicherbereichen (21 . . . 24) aufweist, die jeweils die den Bildpunkten eines Einzelbildes zugeordneten Signalwerte speichern und die gleichzeitig und unabhängig voneinander adressierbar sind und dass die Bildwiedergabeeinheit (27) das Schichtbild punktweise ausgibt und so mit den Speicherbereichen synchronisiert ist, dass jeweils der Schichtbildpunkt wiedergegeben wird, dessen geometrisch zugehörige Bildpunkte gerade aus den Speicherbereichen aufgerufen sind.

6. Vorrichtung nach Anspruch 4, dadurch gekennzeichnet, dass in jeder Strahlenquellenposition ein Röntgenstrahler (21 . . . 24) angeordnet ist und dass die Röntgenstrahler gleichzeitig oder nacheinander einschaltbar sind.

7. Vorrichtung nach Anspruch 6, dadurch gekennzeichnet, dass ein einziger Bildwandler vorgesehen ist und dass zwischen den Röntgenstrahlern und dem Bildwandler (7, 9) ein Raster (8) mit einer der Zahl der Bildpunkte eines Einzelbildes entsprechenden Zahl von Öffnungen angeordnet ist derart, dass jeder Punkt (1, 2, 3, 4) auf dem Bildwandler nur von einem Röntgenstrahler bestrahlt wird.

8. Vorrichtung nach einem der vorhergehenden Ansprüche, dadurch gekennzeichnet, dass vier Strahlenquellen vorgesehen sind, die auf den Eckpunkten eines Quadrates angeordnet sind.

9. Vorrichtung nach Anspruch 4 und Anspruch 8, dadurch gekennzeichnet, dass der Extremwertdekoder vier Schaltungen (31 . . . 34) mit Torwirkung enthält, die mit den Ausgängen von vier Speichern (21 . . . 24) gekoppelt sind, in denen die den Bildpunkten der Einzelbilder zugeordneten Messwerte gespeichert sind, und dass für je zwei dieser Schaltungen (31 und 32 bzw. 33 und 34) ein Vergleicher (35 bzw. 37) vorgesehen ist, der die Eingangssignale dieser Schaltungen miteinander vergleicht und die Schaltungen entsprechend steuert, dass die Ausgänge dieser beiden Schaltungen miteinander und mit den Eingängen zweier weiterer Schaltungen (46, 47) mit Torwirkung verbunden sind, die durch einen weiteren Vergleicher (45) gesteuert sind, der die Eingangssignale dieser Schaltungen vergleicht, und deren miteinander verbundene Ausgänge den Ausgang des Extremwertdekoders darstellen.

**Claims**

1. A method of generating layer images of an object which is irradiated from a plurality of radiation source positions in order to generate separate single images, for each single image there being stored measurement values which are dependent on the absorption on its image points whilst for each layer image point an image value which corresponds to the absorption is derived from the stored measurement values of the image points which are geometrically associated with the layer image point, characterized in that the image value is formed by the measurement value (values) which corresponds (correspond) to the lowest absorption.

2. A method as claimed in Claim 1, characterized in that the image value associated with a layer image point is subsequently subtracted from the measurement values of the single image points geometrically associated with this layer image point, the measurement values thus calculated being used for generating further layer images.

3. A method as claimed in Claim 1, characterized in that for each layer image point the number of geometrically associated single image points is determined whose measurement values correspond at least approximately to the maximum value of the absorption.

4. A device comprising a radiation source array (11 . . . 14) for generating a plurality of single images of the object (5) from each time one radiation source position, an image converter unit (7, 9) for converting each single image into electrical signals, a storage device (21 . . . 24) for storing the signals associated with the image points of the single images, and means (28; 210 . . . 240; 150) for fetching the signals associated with the image points geometrically associated with a layer image point from the storage device (21 . . . 24) and for applying a value derived therefrom to an image display unit (27), characterized in that the signals fetched from the storage device (21 . . . 24) are applied to the image display unit via an extreme-value decoder (19) whose output signals forms the signal fetched which corresponds to the lowest absorption.

5. A device as claimed in Claim 4, characterized in that the storage device comprises a number of storage sections (21 . . . 24) which corresponds to the number of radiation source positions, each section storing the signal values associated with the image points of a single image, said sections being simultaneously and independently adressable, the layer image being output point-by-point by the image display unit (27) so that it is synchronized with the storage sections, each time the layer image point being displayed whose geometrically associated image points have just been fetched from the storage sections.

6. A device as claimed in Claim 4, characterized in that an X-ray source (21 . . . 24) is arranged in each radiation source position, the X-ray sources being simultaneously or consecutively activatable.

7. A device as claimed in Claim 6, characterized in that there is provided a single image converter, between the X-ray sources and the image converter (7, 9) there being arranged a grid (8) comprising a number of apertures which corresponds to the number of image points of a single image, so that each point (1, 2, 3, 4) on the image converter is irradiated by one X-ray source only.

8. A device as claimed in one of the preceding Claims, characterized in that there are provided four radiation sources which are arranged at the corners of a square.

9. A device as claimed in Claim 4 and Claim 8, characterized in that the extreme-value decoder comprises four circuits (31 . . . 34) which function as gates and which are coupled to the outputs of four memories (21 . . . 24) in which the measurement values are stored which are associated with the image points of the single images, for each time two of these circuits (31 and 32, 33 and 34) there being provided a comparator (35, 37, respectively) which compares the input signals of these circuits and which controls the circuits accordingly, the outputs of said two circuits being connected to one another and to the inputs of two further circuits (46, 47) which function as gates and which are controlled by a further comparator (45) which compares the input signals of these circuits, the interconnected outputs of said further circuits forming the output of the extreme-value decoder.

**Revendications**

1. Procédé pour produire des images tomographiques d'un objet, qui est exposé à partir de plusieurs positions de sources de rayonnement, afin de produire des images individuelles séparées, suivant lequel, pour chaque image individuelle, des valeurs de mesure dépendant de l'absorption dans ses points d'image sont stockées et, pour chaque point d'image tomographique, une valeur d'image correspondant à l'absorption est dérivée des valeurs de mesure stockées des points d'image associés géométriquement au point d'image tomographique, caractérisé en ce que la valeur d'image est formée par la ou les valeurs de mesure qui correspond ou correspondent à la plus faible absorption.

2. Procédé suivant la revendication 1, caractérisé en ce qu'ensuite, la valeur d'image associée à un point d'image tomographique est soustraite des valeurs de mesure des points d'image individuels associés géométriquement à ce point d'image tomographique et que les valeurs de mesure ainsi calculées sont utilisées comme base pour la production d'autres images tomographiques.

3. Procédé suivant la revendication 1, caractérisé en ce que pour chaque point d'image tomographique est déterminé le nombre des points d'image individuels associés géométriquement à celui-ci dont les valeurs de mesure correspondent au moins approximativement à la valeur maximale de l'absorption.

4. Dispositif pourvu d'un montage de sources de rayonnement (11 . . . 14) pour produire plusieurs images individuelles de l'objet (5) à partir chaque fois d'une position de source de rayonnement, comportant un dispositif convertisseur d'image (7, 9) pour convertir chaque image individuelle en signaux électriques, un dispositif de stockage (21 . . . 24) pour stocker les signaux associés aux points d'image des images individuelles, des moyens (28; 210 . . . 240; 150) pour appeler du dispositif de stockage (21 . . . 24) les signaux appartenant aux points d'image associés géométriquement à un point d'image tomographique et pour appliquer une valeur qui en est dérivée à une unité de reproduction d'image (27), caractérisé en ce que les signaux appelés du dispositif de stockage (21 . . . 24) sont appliqués à l'unité de reproduction d'image par l'intermédiaire d'un décodeur de valeur extrême dont le signal de sortie forme le signal appelé qui correspond à l'absorption la plus faible.

5. Dispositif suivant la revendication 4, caractérisé en ce que le dispositif de stockage comporte des domaines de stockage (21 . . . 24) en un nombre égal au nombre de positions de sources de rayonnement, qui stockent chaque fois les valeurs de signaux associées aux points d'image d'une image individuelle et qui sont adressables simultanément et indépendamment les uns des autres et que l'unité de reproduction d'image (27) sort l'image tomographique point par point et est synchronisée avec les domaines de stockage d'une manière telle que chaque fois est reproduit le point d'image tomographique dont les points d'image associés géométriquement sont précisément appelés des domaines de stockage.

6. Dispositif suivant la revendication 4, caractérisé en ce que dans chaque position de source de rayonnement est disposée une source de rayons X (11 . . . 14) et que les sources de rayons X peuvent être enclenchées simultanément ou successivement.

7. Dispositif suivant la revendication 6, caractérisé en ce qu'un seul convertisseur d'image est prévu et qu'entre les sources de rayons X et le convertisseur d'image (7, 9) est disposée une grille (8) comportant un nombre d'ouvertures correspondant au nombre des points d'image d'une image individuelle d'une manière telle que chaque point (1, 2, 3, 4) sur le convertisseur d'image ne soit exposé qu'à une seule source de rayons X.

8. Dispositif suivant l'une quelconque des revendications précédentes, caractérisé en ce que quatre sources de rayonnement qui sont disposées aux coins d'un carré sont prévues.

9. Dispositif suivant les revendications 4 et 8, caractérisé en ce que le décodeur de valeur extrême comporte quatre circuits (31 ... 34) à effet de porte qui sont couplés aux sorties de quatre mémoires (21 ... 24), dans lesquelles les valeurs de mesure associées aux points d'image des images individuelles sont stockées et que, pour chaque fois deux de ces circuits (31 et 32 ou 33 et 34), est prévu un comparateur (35 ou 37), qui compare les signaux d'entrée de ces circuits l'un à l'autre et pilote les circuits de manière correspondante, que les sorties de ces deux circuits sont connectées l'une à l'autre ainsi qu'aux entrées de deux autres circuits (46, 47) à effet de porte, qui sont pilotés par un autre comparateur (45), qui compare les signaux d'entrée de ces circuits et dont les sorties interconnectées représentent la sortie du décodeur de valeur extrême.

Fig.1

Fig.3

Fig.2

0 106 402

Fig.4

Fig.4

15